# EUROPÄISCHE PATENTANMELDUNG

(11) **EP 4 516 118 A2**
(43) Veröffentlichungstag der Anmeldung: **05.03.2025**
(21) Anmeldenummer: 24222324.6
(22) Anmeldetag: 09.08.2017
(51) Int. Cl.: A23L 27/20

(54) **VERWENDUNG VON AMBROCENIDE® ZUR VERSTÄRKUNG EINER MAIGLÖCKCHEN-NOTE**

(62) Teilanmeldung aus: 17754310.5
(71) Anmelder: Symrise AG, 37603 Holzminden Niedersachsen (DE)
(72) Erfinder: Betzer, Marcus, 37603 Holzminden (DE); Lambrecht, Stefan, 37619 Hehlen (DE); Eh, Marcus, 37603 Holzminden (DE)
(74) Vertreter: Eisenführ Speiser

(57) **Zusammenfassung**

Die vorliegende Erfindung betrifft primär die Verwendung von einem oder mehreren Stereoisomer(en) der Verbindung der Formel (I) wie hierin beschrieben zum Modifizieren und/oder Verstärken des Geruchseindrucks eines oder mehrerer Geruchsstoffs/Geruchsstoffe mit einer Maiglöckchen-Note. Die Erfindung betrifft zudem eine neue Mischung umfassend oder bestehend aus einem oder mehreren Stereoisomer(en) der Verbindung der Formel (I) wie hierin beschrieben und zwei oder mehr Stereoisomeren der Verbindung der Formel (II) wie hierin beschrieben mit einem Gewichtsverhältnis der Stereoisomere der Verbindungen der Formel (II) wie hierin beschrieben, Riechstoffkompositionen umfassend oder bestehend aus einer Mischung wie hierin beschrieben, parfümierte Produkte enthaltend Mischungen oder Riechstoffkomposition wie hierin beschrieben sowie ein Verfahren zum Modifizieren und/oder Verstärken der Maiglöckchen-Note einer Verbindung der Formel (II) wie hierin beschrieben bzw. zum Herstellen eines parfümierten Produktes wie hierin beschrieben.

## Beschreibung

Die vorliegende Erfindung betrifft primär die Verwendung von einem oder mehreren Stereoisomer(en) der Verbindung der Formel (I) wie hierin beschrieben zum Modifizieren und/oder Verstärken des Geruchseindrucks eines oder mehrerer Geruchsstoffs/Geruchsstoffe mit einer Maiglöckchen-Note. Die Erfindung betrifft zudem eine neue Mischung umfassend oder bestehend aus einem oder mehreren Stereoisomer(en) der Verbindung der Formel (I) wie hierin beschrieben und zwei oder mehr Stereoisomeren der Verbindung der Formel (II) wie hierin beschrieben mit einem Gewichtsverhältnis der Stereoisomere der Verbindungen der Formel (II) wie hierin beschrieben, Riechstoffkompositionen umfassend oder bestehend aus einer Mischung wie hierin beschrieben, parfümierte Produkte enthaltend Mischungen oder Riechstoffkomposition wie hierin beschrieben sowie ein Verfahren zum Modifizieren und/oder Verstärken der Maiglöckchen-Note einer Verbindung der Formel (II) wie hierin beschrieben bzw. zum Herstellen eines parfümierten Produktes wie hierin beschrieben.

Weitere Aspekte und bevorzugte Ausgestaltungen der vorliegenden Erfindung ergeben sich aus den nachfolgenden Ausführungen, den beigefügten Beispielen sowie insbesondere den beigefügten Patentansprüchen.

Trotz einer Vielzahl bereits vorhandener Riechstoffe besteht in der Parfümindustrie auch weiterhin ein genereller Bedarf an neuen Riechstoffen und Riechstoffkompositionen. So besteht beispielsweise ein Bedarf an Riechstoffen, die in der Lage sind (in Riechstoffkompositionen) neben einer primären Duftnote weitere interessante Noten zu erzeugen und mit ihren neuartigen bzw. originellen olfaktorischen Eigenschaften die Möglichkeiten des Parfümeurs zu erweitern. Insbesondere besteht ein Interesse an Riechstoffen mit Duftnoten, welche in der Lage sind, eine harmonische Kombination mit blumigen und/oder fruchtigen Riechstoffen einzugehen. Vorzugsweise sollte dabei eine Überlagerung der unterschiedlichen geruchlichen Aspekte und Noten erfolgen, um dadurch einen insgesamt komplexen Geruchseindruck zu erzeugen.

Für die Kreation neuartiger Kompositionen besteht ständiger Bedarf an Riechstoffen mit besonderen sensorischen Eigenschaften, die geeignet sind, als Grundlage für die Komposition von neuartigen Parfüms mit komplexem sensorischen Charakter zu dienen.

Die primäre Aufgabe bestand nun darin, Riechstoffe zu finden, die ein interessantes, vorzugsweise komplexes, und originelles sensorisches Profil aufweisen und sich als Riechstoffe für den Einsatz in z.B. der Parfümerie eignen. Es wurden im Rahmen der vorliegenden Erfindung insbesondere Stoffe gesucht, die eine Maiglöckchen-Geruchsnote modifizieren und/oder verstärken können.

Die gesuchten Stoffe sollten die Herstellung von neuartigen Riechstoffkompositionen mit besonderen geruchlichen Noten und Aspekten ermöglichen. Von Vorteil wären dabei solche Stoffe, welche insbesondere zur Kombination mit weiteren Riechstoffen geeignet sind, die die Note Maiglöckchen aufweisen.

Daneben sollten die diese primäre Aufgabe erfüllenden Riechstoffe vorzugsweise über ihre primären, nämlich geruchlichen, Eigenschaften hinaus zusätzliche positive Sekundäreigenschaften besitzen, wie z.B. eine hohe Stabilität unter bestimmten Anwendungsbedingungen, eine hohe Ausgiebigkeit, ein gutes Haftungsvermögen, eine hohe Substantivität oder geruchsverstärkende Eigenschaften (sogenannte Booster- oder Enhancer-Wirkung), und/oder in Kombination mit anderen Riechstoffen deren Natürlichkeit, Frische, Fülle, (Strahl-)Kraft und/oder Ausstrahlung abrunden, so dass sensorisch bemerkenswerte Effekte erzielt werden können.

Erfindungsgemäß gelöst wird die primäre gestellte Aufgabe durch die Verwendung von einem oder mehreren Stereoisomer(en) der Verbindung der Formel (I) 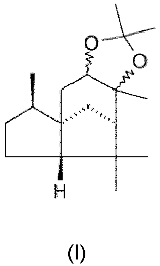 zum Modifizieren und/oder Verstärken, vorzugsweise zum Verstärken, des Geruchseindrucks eines oder mehrerer Geruchsstoffs/Geruchsstoffe mit einer Maiglöckchen-Note.

Im Rahmen der der vorliegenden Erfindung zugrunde liegenden Studie hat sich überraschenderweise gezeigt, dass die Verbindung der Formel (I) (Ambrocenide^{®}, CAS-Nr.: 211299-54-6) hervorragend dafür geeignet ist, die Maiglöckchen-Note verschiedener Maiglöckchen-Riechstoffe zu modifizieren und/oder zu verstärken, insbesondere zu verstärken.

Gemäß der vorliegenden Erfindung können als Verbindung der Formel (I) ein, zwei, drei oder sämtliche der folgenden Diastereomere (Ia) bis (Id) eingesetzt werden, d.h. es können jeweils die einzelnen Diastereomere (Ia) bis (Id) eingesetzt werden oder eine beliebige Mischung dieser Diastereomere.

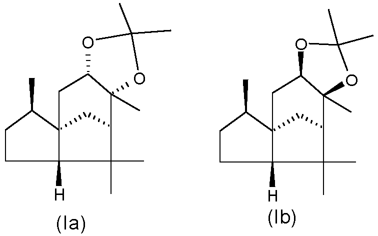

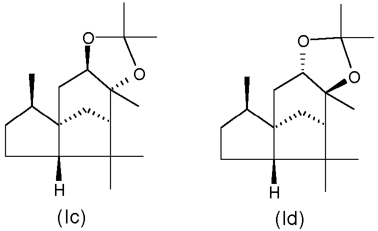

Das hierin für eine Verbindung der Formel (I) Gesagte, insbesondere die hierin beschrieben Vorteile, gilt/gelten ebenso für die einzelnen Diastereomere (Ia) bis (Id) und eine beliebige Mischung dieser Diastereomere (s. oben).

Vorzugsweise betrifft die erfindungsgemäße Verwendung eine Verwendung wie hierin beschrieben, wobei der bzw. die oder ein bzw. mehrere Geruchsstoff(e) ausgewählt ist bzw. sind aus der Gruppe bestehend aus 2-Methyl-3-(4-tert.-butylphenyl)propanal, 2-Methyl-3-(3-tert.-butylphenyl)propanal, 2,5,7,7-Tetramethyloctanal, 4-(1,1-Dimethylethyl)phenylpropanal, 3-(4-Isopropylphenyl)-propanal, Octahydro-8,8-dimethylnaphthalene-2-carboxaldehyd, Octahydro-4,7-methanoindanilydenebutanal, beta-Methyl-3-(1-methylethyl)phenylpropanal, 2-Methyl-3-(3,4-methylendioxyphenyl)propanal, 7-Hydroxy-3,7-dimethyloctanal, 4-(4-Hydroxy-4-methylpentyl)-3-cyclohexen-1-carboxaldehyd, 2,2-Dimethyl-3-(3-methylphenyl)propanol, cis-4-(1-methylethyl)cyclohexanmethanol, 1-(4-Isopropylcyclohexyl)ethanol, 3-Methyl-4-phenylbutan-2-ol, Dimethylphenyl-propanol, 2-Methyl-3-(4-(2-methylpropyl)-phenyl)propanal, 3-(4-Isobutylphenyl)-2-methylpropanal, 3,4-Dioxy(cycloacetonyl)toluol, 3-(1-Ethoxyethoxy)-3,7-dimethyl-1,6-octadien, alpha,alpha-Dimethyl-4-ethylphenyl-propanal, gamma-Methyl-phenylpentanal und Verbindung der Formel (II), 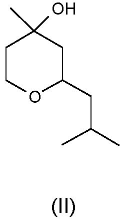 vorzugsweise wobei der Geruchsstoff eine Verbindung der Formel (II) ist.

Insbesondere bevorzugt ist eine Verwendung wie hierin beschrieben, wobei der Geruchsstoff bzw. einer der Geruchsstoffe zwei oder mehr Stereoisomere der Verbindung der Formel (II) umfasst oder aus ihnen besteht 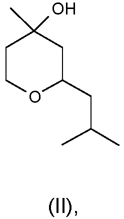 wobei vorzugsweise das Gewichtsverhältnis der Gesamtmenge an Verbindungen der Formel cis-(II)(+) und cis-(II)(-) 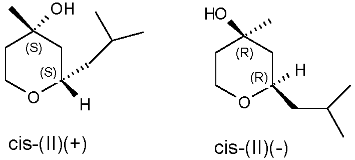 zur Gesamtmenge an Verbindungen der Formel trans-(II)(+) und trans-(II)(-) 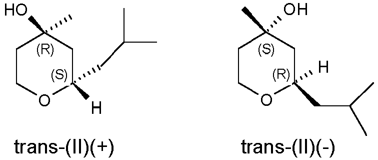 zwischen 65 : 35 und 95 : 5, bevorzugt 70 : 30 und 90 : 10, besonders bevorzugt 75 : 25 und 85 : 15, liegt.

Die erfindungsgemäße Verwendung der Verbindung der Formel (II) wie hierin beschrieben mit einem Isomerenverhältnis in dem vorangehend definierten Bereich ist im Rahmen der vorliegenden Erfindung besonders bevorzugt. Die Verbindung der Formel (II) wie hierin beschrieben mit einem Isomerenverhältnis in dem vorangehend definierten Bereich ist beispielsweise unter dem Handelsnamen Pyranol bekannt. Pyranol ist relativ leicht flüchtig und hat einen weichen blumigen Duft mit einer typischen Note von Maiglöckchen (lily of the valley, muguet), welcher gemäß der vorliegenden Erfindung durch den Zusatz von einem oder mehreren Stereoisomer(en) der Verbindung der Formel (I) überraschenderweise natürlicher und strahlender wirkt. Zudem wirkt der Maiglöckchen-Duft von Pyranol durch den Zusatz von einem oder mehreren Stereoisomer(en) der Verbindung der Formel (I) ausgeprägter (Boost-Effekt) und die Substantivität wird erhöht.

Manche Isomerengemische der Verbindung der Formel (II), beispielsweise als Florol^{®}/Florosa^{®}, werden zum Teil als zu teuer empfunden. Andere Isomerengemische, wie z.B. Pyranol, werden zum Teil gegenüber anderen Isomerengemischen der Verbindung der Formel (II) in Bezug auf seine olfaktorischen Eigenschaften als Geruchsstoff mit weniger Charakter, weniger Strahlung und/oder weniger Haftung des Duftes empfunden. Überraschenderweise führt der Zusatz von einem oder mehreren Stereoisomer(en) der Verbindung der Formel (I) zu einer sensorischen Aufwertung (vgl. hierzu Beispiele unten), d.h. zu mehr Charakter, mehr Strahlung und/oder mehr Haftung des Duftes.

Weiterhin vorteilhaft an der Verwendung von Verbindungen der Formel (II) gegenüber anderen Geruchsstoffen mit einer Maiglöckchen-Note (wie beispielsweise vorangehend aufgelistet) ist, dass die Verbindungen der Formel (II) regulatorisch weniger belastet sind (harmlosere Einstufung/Labelling) und ein besseres Kosten-Leistungs-Verhältnis aufweisen.

Gemäß einer Ausführungsform der erfindungsgemäßen Verwendung ist es bevorzugt, wenn die Gesamtmenge an Verbindungen der Formel cis-(II)(+) und cis-(II)(-) überwiegend - d.h. zu mehr als 50 Gew.-% bezogen auf die Gesamtmenge an Verbindungen der Formel cis-(II)(+) und cis-(II)(-) - Verbindung der Formel cis-(II)(-) umfasst, vorzugsweise wenn sie 60, 70, 80 oder 90 Gew.-% an Verbindung der Formel cis-(II)(-) umfasst, oder wenn sie im Wesentlichen, vorzugsweise zu 100 Gew.-% bezogen auf die Gesamtmenge an Verbindungen der Formel cis-(II)(+) und cis-(II)(-), aus Verbindung der Formel cis-(II)(-) besteht.

Gemäß einer Ausführungsform der erfindungsgemäßen Verwendung ist es bevorzugt, wenn die Gesamtmenge an Verbindungen der Formel cis-(II)(+) und cis-(II)(-) überwiegend - d.h. zu mehr als 50 Gew.-% bezogen auf die Gesamtmenge an Verbindungen der Formel cis-(II)(+) und cis-(II)(-) - Verbindung der Formel cis-(II)(+) umfasst, vorzugsweise wenn sie 60, 70, 80 oder 90 Gew.-% an Verbindung der Formel cis-(II)(+) umfasst, oder wenn sie im Wesentlichen, vorzugsweise zu 100 Gew.-% bezogen auf die Gesamtmenge an Verbindungen der Formel cis-(II)(+) und cis-(II)(-), aus Verbindung der Formel cis-(II)(+) besteht.

Gemäß einem weiteren Aspekt betrifft die vorliegende Erfindung daher auch eine Mischung umfassend oder bestehend aus einem oder mehreren Stereoisomer(en) der Verbindung der Formel (I) 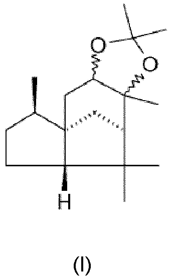 und zwei oder mehr Stereoisomeren der Verbindung der Formel (II) 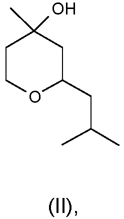 wobei das Gewichtsverhältnis der Gesamtmenge an Verbindungen der Formel cis-(II)(+) und cis-(II)(-) 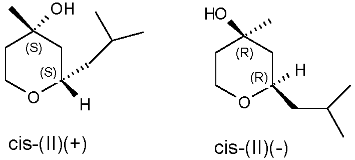 zur Gesamtmenge an Verbindungen der Formel trans-(II)(+) und trans-(II)(-) 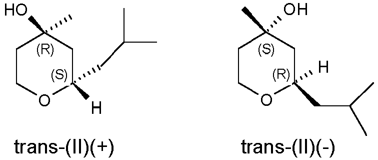 zwischen 65 : 35 und 95 : 5, bevorzugt 70 : 30 und 90 : 10, besonders bevorzugt 75 : 25 und 85 : 15, liegt.

Bevorzugt ist dabei eine Mischung wie hierin beschrieben, wobei das Gewichtsverhältnis der Gesamtmenge an Stereoisomer(en) der Verbindung der Formel (I) 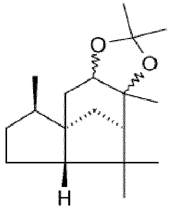 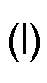 zur Gesamtmenge an Stereoisomeren der Verbindung der Formel (II) 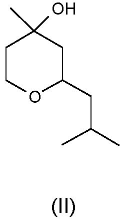 zwischen 1 : 12000 und 1 : 0,5, vorzugsweise zwischen 1 : 600 und 1 : 0,7, besonders bevorzugt zwischen 1 : 100 und 1 : 1, liegt,
und/oder
wobei die Gesamtmenge an Stereoisomer(en) der Verbindung der Formel (I) ausreicht, um den Geruchseindruck der zwei oder mehr Stereoisomeren der Verbindung der Formel (II) zu modifizieren und/oder zu verstärken, vorzugsweise zu verstärken.

Gemäß einem weiteren Aspekt betrifft die vorliegende Erfindung außerdem ein Verfahren zum Modifizieren und/oder Verstärken, vorzugsweise zum Verstärken, der Maiglöckchen-Note einer Verbindung der Formel (II), umfassend oder bestehend aus folgenden Schritten:
i) Bereitstellen von einem oder mehreren Stereoisomer(en) der Verbindung der Formel (I) 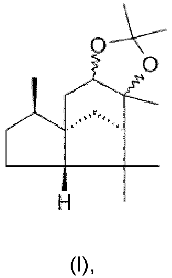
ii) Bereitstellen von zwei oder mehr Stereoisomeren der Verbindung der Formel (II) 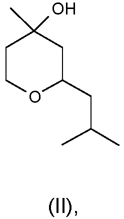 wobei das Gewichtsverhältnis der Gesamtmenge an Verbindungen der Formel cis-(II)(+) und cis-(II)(-) 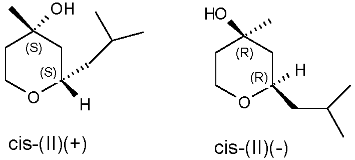 zur Gesamtmenge an Verbindungen der Formel trans-(II)(+) und trans-(II)(-) 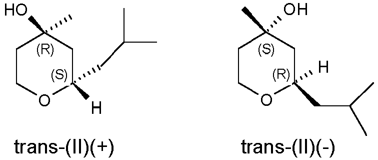 zwischen 65 : 35 und 95 : 5, bevorzugt 70 : 30 und 90 : 10, besonders bevorzugt 75 : 25 und 85 : 15, liegt,
iii) Mischen der in den Schritten i) und ii) bereitgestellten Verbindungen, vorzugsweise wobei die Menge des einen oder der mehreren Stereoisomers/Stereoisomere der Verbindung der Formel (I) ausreicht, um die Maiglöckchen-Note der Verbindung der Formel (II) zu modifizieren und/oder zu verstärken, vorzugsweise zu verstärken.

Bevorzugt ist ein erfindungsgemäßes Verfahren wie hierin beschrieben, wobei das Gewichtsverhältnis der Gesamtmenge an Stereoisomer(en) der Verbindung der Formel (I) 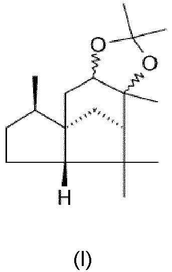 zur Gesamtmenge an Stereoisomeren der Verbindung der Formel (II) 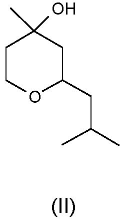 zwischen 1 : 12000 und 1 : 0,5, vorzugsweise zwischen 1 : 600 und 1 : 0,7, besonders bevorzugt zwischen 1 : 10 und 1 : 1, liegt.

Ein weiterer Aspekt der vorliegenden Erfindung betrifft eine Mischung, vorzugsweise eine Mischung wie hierin beschrieben, herstellbar durch ein Verfahren umfassend oder bestehend aus folgenden Schritten:
i) Bereitstellen von einem oder mehreren Stereoisomer(en) der Verbindung der Formel (I) 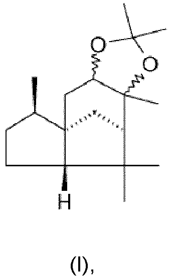
ii) Bereitstellen von zwei oder mehr Stereoisomeren der Verbindung der Formel (II) 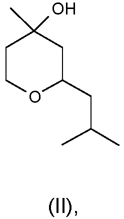 wobei das Gewichtsverhältnis der Gesamtmenge an Verbindungen der Formel cis-(II)(+) und cis-(II)(-) 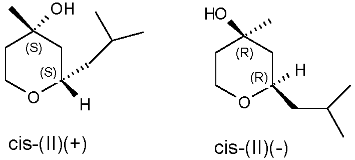 zur Gesamtmenge an Verbindungen der Formel trans-(II)(+) und trans-(II)(-) 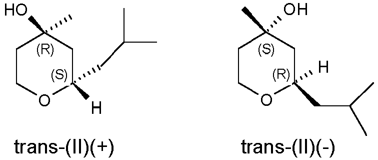 zwischen 65 : 35 und 95 : 5, bevorzugt 70 : 30 und 90 : 10, besonders bevorzugt 75 : 25 und 85 : 15, liegt,
iii) Mischen der in den Schritten i) und ii) bereitgestellten Verbindungen.

Gemäß einem weiteren Aspekt betrifft die vorliegende Erfindung zudem eine Riechstoffkomposition, vorzugsweise Parfümöl, umfassend oder bestehend aus einer Mischung wie hierin beschrieben und vorzugsweise zudem einem oder mehreren zusätzlichen Riechstoffen,
vorzugsweise wobei der zusätzliche bzw. einer, mehrere oder sämtliche der zusätzlichen Riechstoffe ausgewählt ist bzw. sind aus der Gruppe bestehend aus 3-(4-Methyl-1-cyclohex-3-enyl)-butanal, 4-(4-Hydroxyphenyl) butan-2-on, (E)-4-(2,6,6-Trimethyl-1-cyclohex-2-enyl)but-3-en-2-on, (E)-4-(2,6,6-Trimethyl-1-cyclohex-2-enyl)but-3-en-2-on, (E)-1-(2,6,6-trimethyl-cyclohexen-1-yl)pent-1-en-3-on, (E)-4-[(1S)-1 ,2,6,6-tetramethylcyclohex-2-en-1-yl]-but-3-en-2-on, 1-(2,6,6-Trimethyl-1-cyclohex-2-enyl)pent-1-en-3-on, [(Z)-Hex-3-enyl] methyl carbonat, 3-[(Z)-Hex-3-enoxy]propannitril, 1-(2,3,8,8-Tetramethyl-1,3,4,5,6,7-hexa-hydronaphthalin-2-yl)ethanon, spiro[1,3-dioxolan-2,5'-(4',4',8',8'-tetramethyl-hexahydro-3',9'-methanonaphthalin)], [3R-(3α,3aβ,6β,7β,8aα)]-octa-hydro-6-methoxy-3,6,8,8-tetramethyl-1H-3a,7-methanoazulen,[3R-(3α,3aβ,7β,8aα)] - 1-(2,3,4,7,8,8a-hexahydro-3,6,8,8-tetramethyl-1H-3a,7-methano-azulen-5-yl) ethan-1-on, 1-(2,2,6-Trimethyl-cyclohexyl) hexan-3-ol, 6,6-Dimethoxy-2,5,5-trimethylhex-2-en, 2,6-Dimethyloct-7-en-2-ol, 3,7-Dimethylocta-1,6-dien-3-ol, (3,7-Dimethylocta-1,6-dien-3-yl)acetat, (4-Methyl-1-propan-2-yl-1-cyclohex-2-enyl) acetat, (8E)-Cyclohexadec-8-en-1-on, 16-Oxacyclohexa-decan-1-on, 1,3,4,6,7,8-Hexahydro-4,6,6,7,8,8-hexamethyl-cyclopenta(g)-2-benzopyran, Ethoxymethoxy-cyclododecan, 1,1,2,3,3-Pentamethyl-2,5,6,7-tetrahydroinden-4-on, 1-(2,3,8,8-Tetramethyl-1,3,4,5,6,7-hexa-hydronaphthalin-2-yl)ethanon.

Bevorzugt ist dabei eine Riechstoffkomposition wie hierin beschrieben, wobei die Gesamtmenge an Stereoisomer(en) der Verbindung der Formel (I) 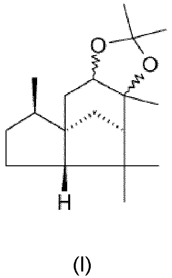 ausreicht, um den Geruchseindruck der zwei oder mehr Stereoisomeren der Verbindung der Formel (II) 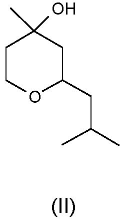 zu modifizieren und/oder zu verstärken, vorzugsweise zu verstärken.

Weiter bevorzugt ist eine erfindungsgemäße Riechstoffkomposition wie hierin beschrieben, vorzugsweise Parfümöl, wobei die Gesamtmenge an Stereoisomer(en) der Verbindung der Formel (I) 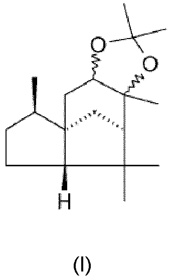 bezogen auf das Gesamtgewicht der Riechstoffkomposition 0,0001 bis 10 Gew.-%, vorzugsweise 0,001 bis 5 Gew.-%, besonders bevorzugt 0,01 bis 1 Gew.-%, beträgt.

Ein weiterer Aspekt der vorliegenden Erfindung betrifft ein parfümiertes Produkt, enthaltend eine erfindungsgemäße Mischung wie hierin beschrieben, oder vorzugsweise eine erfindungsgemäße Riechstoffkomposition wie hierin beschrieben, vorzugsweise ein Parfümöl, in einer sensorisch wirksamen Menge, wobei der Anteil der Mischung bzw. Riechstoffkomposition bezogen auf das Gesamtgewicht des Produktes vorzugsweise im Bereich von 0,01 bis 100 Gew.-%, vorzugsweise 0,02 bis 50 Gew.-%, besonders bevorzugt 0,1 bis 20 Gew.-%, liegt,
vorzugsweise wobei das Produkt ausgewählt ist aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

Ein weiterer Aspekt der vorliegenden Erfindung betrifft zudem ein Verfahren zum Herstellen eines parfümierten Produktes, vorzugsweise eines erfindungsgemäßen Produktes wie hierin beschrieben, umfassend folgende Schritte:
i) Bereitstellen einer erfindungsgemäßen Mischung wie hierin beschrieben oder einer erfindungsgemäßen Riechstoffkomposition wie hierin beschrieben,
ii) Bereitstellen eines oder mehrerer weiterer Bestandteile des herzustellenden parfümierten Produktes, und
iii) Inkontaktbringen oder Mischen der in Schritt ii) bereitgestellten weiteren Bestandteile mit einer sensorisch wirksamen Menge der in Schritt i) bereitgestellten Bestandteile.

Für die vorstehend beschriebenen erfindungsgemäßen Aspekte gilt vorzugsweise jeweils das oben im Zusammenhang mit einer erfindungsgemäßen Verwendung Gesagte entsprechend und ebenso gilt das im Zusammenhang mit den vorstehend beschriebenen erfindungsgemäßen Aspekten Gesagte entsprechend für die erfindungsgemäße Verwendung. Des Weiteren sind die hierin beschriebenen Ausführungsformen, soweit technisch sinnvoll, beliebig miteinander kombinierbar.

Die vorliegende Erfindung wird anhand der nachfolgenden Beispiele näher erläutert. Sofern nichts Anderes angegeben ist, beziehen sich dabei sämtliche Angaben auf das Gewicht.

### Beispiele:

### Bevorzugte Mischungen von Pyranol mit Ambrocenide^{®}

### Mischung 1

| | |
|---|---|
| PYRANOL | 1.000,00 |
| AMBROCENIDE^{®} | 0,1 |

Effekt: Die Maiglöckchen Note des Pyranols wird verstärkt und prägnanter was mit einer Höherwertigkeit einher geht.

### Mischung 2

| | |
|---|---|
| PYRANOL | 1.000,00 |
| AMBROCENIDE^{®} | 1 |

Effekt: Die Maiglöckchen Note des Pyranols wird substantiver und natürlicher, was die Mischung 2 wertvoller erscheinen lässt.

### Parfümöle 1

| | **A** | **B** | **C** | **D** |
|---|---|---|---|---|
| ALDEHYD C14 SOG | 3,00 | 3 | 3 | 3 |
| AMBROXIDE | 15 | 15 | 15 | 15 |
| BENZOE SIAM ABS. | 30 | 30 | 30 | 30 |
| BENZYLACETAT FG | 2 | 2 | 2 | 2 |
| BHT JONOL | 2 | 2 | 2 | 2 |
| CASHMERAN | 20 | 20 | 20 | 20 |
| CUMARIN | 7 | 7 | 7 | 7 |
| DECALACTON GAMMA | 4 | 4 | 4 | 4 |
| DIPROPYLENGLYCOL | 69,5 | 69,5 | 69,5 | 69,5 |
| EBANOL | 15 | 15 | 15 | 15 |
| ETHYLENBRASSYLAT | 150 | 150 | 150 | 150 |
| ETHYLMALTOL | 10 | 10 | 10 | 10 |
| FLOROSA | 120 | | | |
| PYRANOL | | 120 | | |
| HEXENOL CIS-3 10% DPG | 8 | 8 | 8 | 8 |
| HEXENYLACETAT CIS-3 | 1 | 1 | 1 | 1 |
| INDOL FF 1% DPG | 5 | 5 | 5 | 5 |
| ISO E SUPER | 320 | 320 | 320 | 320 |
| ISOEUGENOL 10% DPG | 1,5 | 1,5 | 1,5 | 1,5 |
| JASMIN ABS. SAMBAC REFA 50% BB | 1,5 | 1,5 | 1,5 | 1,5 |
| JASMON CIS | 1 | 1 | 1 | 1 |
| METHYLANTHRANILAT 10% DPG | 0,5 | 0,5 | 0,5 | 0,5 |
| MUSCENONE | 7 | 7 | 7 | 7 |
| MUSCONE | 7 | 7 | 7 | 7 |
| NORLIMBANOL | 15 | 15 | 15 | 15 |
| POLYSANTOL (MYSANTOL) | 30 | 30 | 30 | 30 |
| RED BERRIES EXTRACT | 5 | 5 | 5 | 5 |
| SANDRANOL^{®} | 150 | 150 | 150 | 150 |
| Mischung 1 | | | 120 | |
| Mischung 2 | | | | 120 |
| TOTAL | 1000 | 1000 | 1000 | 1000 |

Parfümöl A: Standard-Riechstoffkomposition mit Florosa^{®} als Maiglöckchen-Duftstoff

Parfümöl B: Florosa^{®} aus Parfümöl A wird durch Pyranol ersetzt. Der Duft von Parfümöl B ist im Vergleich zu Parfümöl A weniger floral, weniger strahlend und verliert an Komplexität.

Parfümöl C: Florosa^{®} aus Parfümöl A wird durch Mischung 1 (wie oben beschrieben) ersetzt. Der Duft von Parfümöl C ist im Vergleich zu Parfümöl B strahlender, hat mehr Körper und hat eine bessere Hedonik.

Parfümöl D: Florosa^{®} aus Parfümöl A wird durch Mischung 2 (wie oben beschrieben) ersetzt. Der Duft von Parfümöl D ist im Vergleich zu Parfümöl B stärker und substantiver. Darüber hinaus ist Parfümöl D auch strahlender und komplexer und insgesamt performanter als Parfümöl A.

### Parfümöle 2

| | **E** | **F** | **G** |
|---|---|---|---|
| AMAROCIT^{®} | 10 | 10 | 10 |
| AMBRETTOLIDE | 1,5 | 1,5 | 1,5 |
| AMBROXIDE | 0,5 | 0,5 | 0,5 |
| AMYRISOEL | 2,5 | 2,5 | 2,5 |
| AURELIONE^{®} | 25 | 25 | 25 |
| BENZYLSALICYLAT | 9 | 9 | 9 |
| BERGAMOTTOEL BERGAPTENFREI | 40 | 40 | 40 |
| CITRAL FF | 1,5 | 1,5 | 1,5 |
| CITRONELLOL L | 1,5 | 1,5 | 1,5 |
| CLARITONE^{®} | 5 | 5 | 5 |
| DIMETHYLBENZYLCARBINYLBUTYRAT 10% DPG | 1 | 1 | 1 |
| ETHYLENBRASSYLAT | 50 | 50 | 50 |
| FLOROPAL 10% DPG | 3 | 3 | 3 |
| FLOROSA | 90 | | |
| GLOBALIDE^{®} | 50 | 50 | 50 |
| HEDION HC/30 | 400 | 400 | 400 |
| HELIOTROPIN/PIPERONAL | 20 | 20 | 20 |
| HEXYLZIMTALDEHYD ALPHA 10% DPG | 1 | 1 | 1 |
| ISO E SUPER | 200 | 200 | 200 |
| ISOBORNYLCYCLOHEXANOL | 10 | 10 | 10 |
| ISORALDEIN 95 | 30 | 30 | 30 |
| LINALOOL | 12 | 12 | 12 |
| MAGNOLAN | 23 | 23 | 23 |
| MANDARINENOEL ENTF. | 6 | 6 | 6 |
| SANDELHOLZOEL AUSTRALIEN PUR | 7,5 | 7,5 | 7,5 |
| Mischung 1 | | 90 | |
| Mischung 2 | | | 90 |
| TOTAL | 1000 | 1000 | 1000 |

Parfümöl E: Standard-Riechstoffkomposition mit Florosa^{®} als Maiglöckchen-Duftstoff

Parfümöl F: Florosa^{®} wird durch Mischung 1 (wie oben beschrieben) ersetzt; die olfaktorische Verbesserung des Duftes ist gering, allerdings können durch die Ersetzung von Florosa^{®} durch Mischung 1 Rohmaterialkosten eingespart werden.

Parfümöl G: Florosa^{®} wird durch Mischung 2 (wie oben beschrieben) ersetzt; der Duft der Riechstoffkomposition G wirkt stärker, substantiver und hat mehr floralen Charakter als der der Kompositionen E und F.

### Parfümierte Produkte

### Waschpulver:

| | |
|---|---|
| AGRUMEX LC | 150 |
| AMBERWOOD^{®} F | 12 |
| BENZYLACETON | 50 |
| CITRONELLOL 950 | 2 |
| CITRONITRIL | 2 |
| CYCLAMENALDEHYD | 1 |
| DIHYDROMYRCENOL | 200 |
| DIPROPYLENGLYCOL | 3 |
| GERANIOL SUPRA | 1 |
| HERBYLPROPIONAT | 80 |
| HEXYLACETAT | 45 |
| IONON ALPHA | 2 |
| IONON BETA | 6 |
| ISO E SUPER | 250 |
| ISODAMASCON^{®} | 1 |
| LINALOOL | 20 |
| MACROLIDE^{®} SUPRA | 10 |
| MAGNOLAN | 2 |
| MANZANATE | 1 |
| NEROLIONE 10% DPG | 2 |
| ORANGENOEL | 15 |
| PHENIRAT^{®} | 50 |
| PHENYLACETALDEHYD DIMETHYLACETAL | 1 |
| PYROPRUNAT | 2 |
| ROSENOXID | 1 |
| SANDRANOL^{®} | 20 |
| UNDECAVERTOL | 5 |
| VERTOCITRAL | 1 |
| YSAMBER^{®} K | 5 |
| Mischung 1 | 60 |

Das Parfümöl verleiht dem Waschpulver den typischen Geruch, den man von diesem Produkt erwartet. Es überträgt die Botschaft von Sauberkeit und Frische. In diesem Fall startet der Duft mit einer Kombination von Aldehyden und Citrus, die die Frische widergeben. Gefolgt vom blumigen Herze, welches den pflegenden Charakter darstellt. Die Basisnote besteht aus lang haftenden Komponenten, die einen holzigen, ambrierten und moschusartigen Charakter haben, um auch noch nach Tagen den Eindruck von frischer Wäsche zu haben.

### Shampoo:

| | |
|---|---|
| AGRUMEX HC | 80 |
| ALDEHYD C14 SOG | 50 |
| ALDEHYD C16 SOG. | 1 |
| AMBROXIDE | 4 |
| ANISALDEHYD REIN | 16 |
| APPLE GREEN AROMABASE | 15 |
| APRIFLOREN^{®} | 1 |
| BERGAMOTTOEL ECHT | 20 |
| CITRONELLOL 950 | 40 |
| CITRONELLYLACETAT EXTRA | 10 |
| DECALACTON GAMMA | 5 |
| DIHYDROMYRCENOL | 100 |
| DIPROPYLENGLYCOL | 8 |
| ETHYLVANILLIN 10% DPG | 4 |
| EUGENOL NAT. | 3 |
| FRAMBINON^{®} 10% DPG | 6 |
| GERANYLACETAT 60 | 20 |
| GERANYLTIGLINAT | 5 |
| GLOBALIDE^{®} | 25 |
| HEDION | 60 |
| HEXENAL TRANS-2 10% DPG | 6 |
| HEXENYLACETAT CIS-3 10% DPG | 9 |
| HEXYLACETAT | 50 |
| HEXYLSALICYLAT | 15 |
| INDOFLOR^{®} KRIST. 10% DPG | 2 |
| ISO E SUPER | 20 |
| ISORALDEIN 70 | 15 |
| JASMIN 61 TYPE BASE | 20 |
| LINALOOL | 60 |
| LINALYLACETAT | 150 |
| PHENIRAT^{®} | 50 |
| ROSE DE MAI-BASE | 30 |
| ROSENOXID L | 5 |
| SANDRANOL^{®} | 15 |
| VERTOCITRAL | 10 |
| Mischung 2 | 70 |

Das Parfümöl transportiert als Duft die Erwartungen, die man an das Produkt hat. In diesem Fall, einem Shampoo, erwartet man Reinigung und Pflege. Dies wird durch den Duft vermittelt. Eine saubere Apfel/Citrus Kombination spiegelt den reinigenden Teil wider, während das Herz des Duftes (Blumen- mit Fruchtnoten) den pflegenden Aspekt darstellt. Abgerundet wird das Dufterlebnis durch warme, holzige und moschusartige Komponenten.

## Patentansprüche

1. Verwendung von einem oder mehreren Stereoisomer(en) der Verbindung der Formel (I) 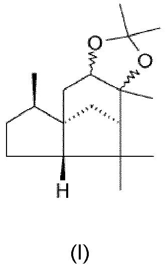 zum Modifizieren und/oder Verstärken, vorzugsweise zum Verstärken, des Geruchseindrucks eines oder mehrerer Geruchsstoffs/Geruchsstoffe mit einer Maiglöckchen-Note.

2. Verwendung nach Anspruch 1, wobei der bzw. die oder ein bzw. mehrere Geruchsstoff(e) ausgewählt ist bzw. sind aus der Gruppe bestehend aus 2-Methyl-3-(4-tert.-butylphenyl)propanal, 2-Methyl-3-(3-tert.-butylphenyl)propanal, 2,5,7,7-Tetramethyloctanal, 4-(1,1-Dimethylethyl)phenylpropanal, 3-(4-Isopropylphenyl)-propanal, Octahydro-8,8-dimethylnaphthalene-2-carboxaldehyd, Octahydro-4,7-methanoindanilydenebutanal, beta-Methyl-3-(1-methylethyl)phenylpropanal, 2-Methyl-3-(3,4-methylendioxyphenyl)propanal, 7-Hydroxy-3,7-dimethyloctanal, 4-(4-Hydroxy-4-methylpentyl)-3-cyclohexen-1-carboxaldehyd, 2,2-Dimethyl-3-(3-methylphenyl)propanol, cis-4-(1-methylethyl)cyclohexanmethanol, 1-(4-Isopropylcyclohexyl)ethanol, 3-Methyl-4-phenylbutan-2-ol, Dimethylphenyl-propanol, 2-Methyl-3-(4-(2-methylpropyl)phenyl)propanal, 3-(4-Isobutylphenyl)-2-methylpropanal, 3,4-Dioxy(cycloacetonyl)toluol, 3-(1-Ethoxyethoxy)-3,7-dimethyl-1,6-octadien, alpha,alpha-Dimethyl-4-ethylphenylpropanal, gamma-Methyl-phenylpentanal und Verbindung der Formel (II), 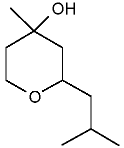 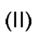 vorzugsweise wobei der Geruchsstoff eine Verbindung der Formel (II) ist.

3. Verwendung nach Anspruch 1 oder 2, wobei der Geruchsstoff bzw. einer der Geruchsstoffe zwei oder mehr Stereoisomere der Verbindung der Formel (II) umfasst oder aus ihnen besteht 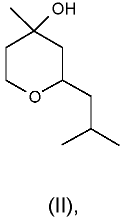 wobei vorzugsweise das Gewichtsverhältnis der Gesamtmenge an Verbindungen der Formel cis-(II)(+) und cis-(II)(-) 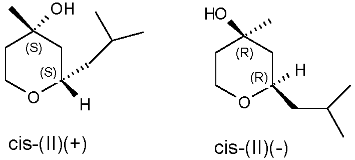 zur Gesamtmenge an Verbindungen der Formel trans-(II)(+) und trans-(II)(-) 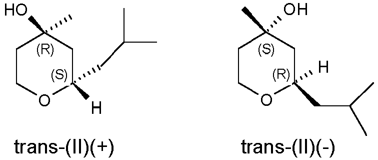 zwischen 65 : 35 und 95 : 5, bevorzugt 70 : 30 und 90 : 10, besonders bevorzugt 75 : 25 und 85 : 15, liegt.

4. Mischung umfassend oder bestehend aus einem oder mehreren Stereoisomer(en) der Verbindung der Formel (I) 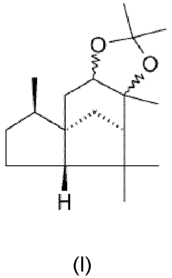 und zwei oder mehr Stereoisomeren der Verbindung der Formel (II) 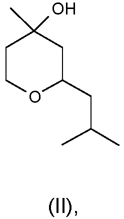 wobei das Gewichtsverhältnis der Gesamtmenge an Verbindungen der Formel cis-(II)(+) und cis-(II)(-) 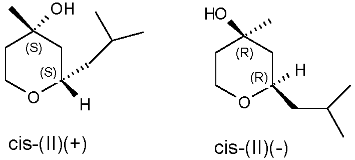 zur Gesamtmenge an Verbindungen der Formel trans-(II)(+) und trans-(II)(-) 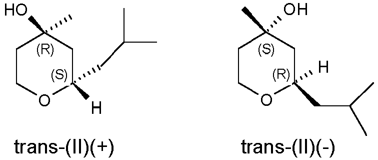 zwischen 65 : 35 und 95 : 5, bevorzugt 70 : 30 und 90 : 10, besonders bevorzugt 75 : 25 und 85 : 15, liegt.

5. Mischung nach Anspruch 4, wobei das Gewichtsverhältnis der Gesamtmenge an Stereoisomer(en) der Verbindung der Formel (I) 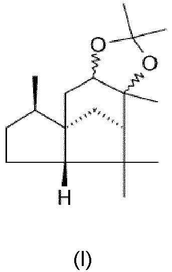 zur Gesamtmenge an Stereoisomeren der Verbindung der Formel (II) 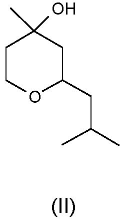 zwischen 1 : 12000 und 1 : 0,5, vorzugsweise zwischen 1 : 600 und 1 : 0,7, besonders bevorzugt zwischen 1 : 100 und 1 : 1, liegt,
und/oder
wobei die Gesamtmenge an Stereoisomer(en) der Verbindung der Formel (I) ausreicht, um den Geruchseindruck der zwei oder mehr Stereoisomeren der Verbindung der Formel (II) zu modifizieren und/oder zu verstärken, vorzugsweise zu verstärken.

6. Verfahren zum Modifizieren und/oder Verstärken, vorzugsweise zum Verstärken, der Maiglöckchen-Note einer Verbindung der Formel (II), umfassend oder bestehend aus folgenden Schritten:
i) Bereitstellen von einem oder mehreren Stereoisomer(en) der Verbindung der Formel (I) 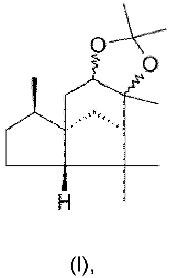
ii) Bereitstellen von zwei oder mehr Stereoisomeren der Verbindung der Formel (II) 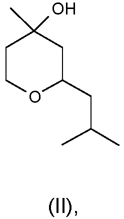 wobei das Gewichtsverhältnis der Gesamtmenge an Verbindungen der Formel cis-(II)(+) und cis-(II)(-) 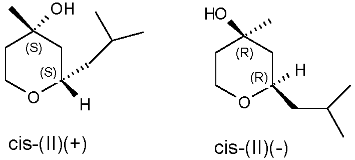 zur Gesamtmenge an Verbindungen der Formel trans-(II)(+) und trans-(II)(-) 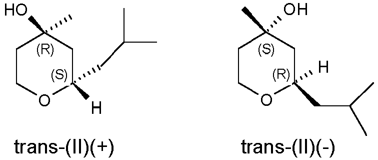 zwischen 65 : 35 und 95 : 5, bevorzugt 70 : 30 und 90 : 10, besonders bevorzugt 75 : 25 und 85 : 15, liegt,
iii) Mischen der in den Schritten i) und ii) bereitgestellten Verbindungen, vorzugsweise wobei die Menge des einen oder der mehreren Stereoisomers/Stereoisomere der Verbindung der Formel (I) ausreicht, um die Maiglöckchen-Note der Verbindung der Formel (II) zu modifizieren und/oder zu verstärken, vorzugsweise zu verstärken.

7. Verfahren nach Anspruch 6, wobei das Gewichtsverhältnis der Gesamtmenge an Stereoisomer(en) der Verbindung der Formel (I) 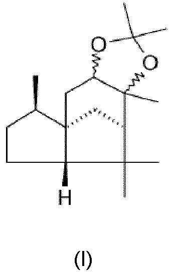 zur Gesamtmenge an Stereoisomeren der Verbindung der Formel (II) 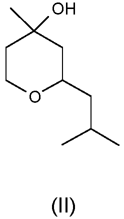 zwischen 1 : 12000 und 1 : 0,5, vorzugsweise zwischen 1 : 600 und 1 : 0,7, besonders bevorzugt zwischen 1 : 10 und 1 : 1, liegt.

8. Mischung, vorzugsweise Mischung nach einem der Ansprüche 4 oder 5, herstellbar durch ein Verfahren umfassend oder bestehend aus folgenden Schritten:
i) Bereitstellen von einem oder mehreren Stereoisomer(en) der Verbindung der Formel (I) 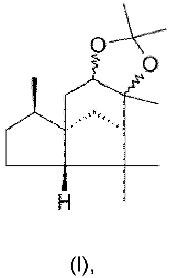
ii) Bereitstellen von zwei oder mehr Stereoisomeren der Verbindung der Formel (II) 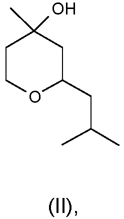 wobei das Gewichtsverhältnis der Gesamtmenge an Verbindungen der Formel cis-(II)(+) und cis-(II)(-) 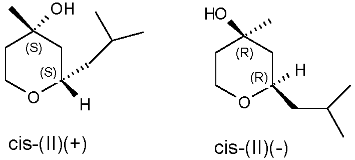 zur Gesamtmenge an Verbindungen der Formel trans-(II)(+) und trans-(II)(-) 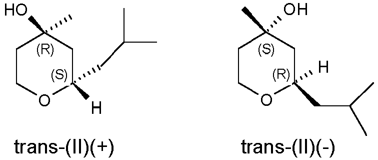 zwischen 65 : 35 und 95 : 5, bevorzugt 70 : 30 und 90 : 10, besonders bevorzugt 75 : 25 und 85 : 15, liegt,
iii) Mischen der in den Schritten i) und ii) bereitgestellten Verbindungen.

9. Riechstoffkomposition, vorzugsweise Parfümöl, umfassend oder bestehend aus einer Mischung nach einem der Ansprüche 4, 5 oder 8 und vorzugsweise zudem einem oder mehreren zusätzlichen Riechstoffen,
vorzugsweise wobei der zusätzliche bzw. einer, mehrere oder sämtliche der zusätzlichen Riechstoffe ausgewählt ist bzw. sind aus der Gruppe bestehend aus 3-(4-Methyl-1-cyclohex-3-enyl)-butanal, 4-(4-Hydroxyphenyl) butan-2-on, (E)-4-(2,6,6-Trimethyl-1-cyclohex-2-enyl)but-3-en-2-on, (E)-4-(2,6,6-Trimethyl-1-cyclohex-2-enyl)but-3-en-2-on, (E)-1-(2,6,6-trimethyl-cyclohexen-1-yl)pent-1-en-3-on, (E)-4-[(1S)-1,2,6,6-tetramethylcyclohex-2-en-1-yl]-but-3-en-2-on, 1-(2,6,6-Trimethyl-1-cyclohex-2-enyl)pent-1-en-3-on, [(Z)-Hex-3-enyl] methyl carbonat, 3-[(Z)-Hex-3-enoxy]propannitril, 1-(2,3,8,8-Tetramethyl-1,3,4,5,6,7-hexa-hydronaphthalin-2-yl)ethanon, spiro[1,3-dioxolan-2,5'-(4',4',8',8'-tetramethyl-hexahydro-3',9'-methanonaphthalin)], [3R-(3α,3aβ,6β,7β,8aα)]-octa-hydro-6-methoxy-3,6,8,8-tetramethyl-1H-3a,7-methanoazulen,[3R-(3α,3aβ,7β,8aα)] -1-(2,3,4,7,8,8a-hexahydro-3,6,8,8-tetramethyl-1H-3a,7-methano-azulen-5-yl) ethan-1-on, 1-(2,2,6-Trimethyl-cyclohexyl) hexan-3-ol, 6,6-Dimethoxy-2,5,5-trimethylhex-2-en, 2,6-Dimethyloct-7-en-2-ol, 3,7-Dimethylocta-1,6-dien-3-ol, (3,7-Dimethylocta-1,6-dien-3-yl)acetat, (4-Methyl-1-propan-2-yl-1-cyclohex-2-enyl) acetat, (8E)-Cyclohexadec-8-en-1-on, 16-Oxacyclohexa-decan-1-on, 1,3,4,6,7,8-Hexahydro-4,6,6,7,8,8-hexamethyl-cyclopenta(g)-2-benzopyran, Ethoxymethoxy-cyclododecan, 1,1,2,3,3-Pentamethyl-2,5,6,7-tetrahydroinden-4-on, 1-(2,3,8,8-Tetramethyl-1,3,4,5,6,7-hexa-hydronaphthalin-2-yl)ethanon.

10. Riechstoffkomposition nach Anspruch 9, wobei die Gesamtmenge an Stereoisomer(en) der Verbindung der Formel (I) 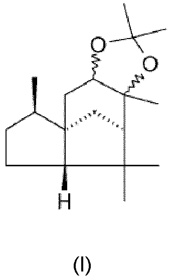 ausreicht, um den Geruchseindruck der zwei oder mehr Stereoisomeren der Verbindung der Formel (II) 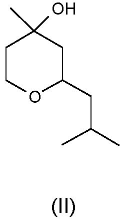 zu modifizieren und/oder zu verstärken, vorzugsweise zu verstärken.

11. Riechstoffkomposition nach einem der Ansprüche 9 oder 10, vorzugsweise Parfümöl, wobei die Gesamtmenge an Stereoisomer(en) der Verbindung der Formel (I) 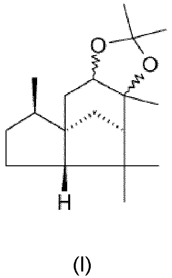 bezogen auf das Gesamtgewicht der Riechstoffkomposition 0,0001 bis 10 Gew.-%, vorzugsweise 0,001 bis 5 Gew.-%, besonders bevorzugt 0,01 bis 1 Gew.-%, beträgt.

12. Parfümiertes Produkt, enthaltend eine Mischung nach einem der Ansprüche 4, 5 oder 8, oder vorzugsweise eine Riechstoffkomposition nach einem der Ansprüche 9 bis 11, vorzugsweise ein Parfümöl, in einer sensorisch wirksamen Menge, wobei der Anteil der Mischung bzw. Riechstoffkomposition bezogen auf das Gesamtgewicht des Produktes vorzugsweise im Bereich von 0,01 bis 100 Gew.-%, vorzugsweise 0,02 bis 50 Gew.-%, besonders bevorzugt 0,1 bis 20 Gew.-%, liegt,
vorzugsweise wobei das Produkt ausgewählt ist aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

13. Verfahren zum Herstellen eines parfümierten Produktes, vorzugsweise eines Produktes nach Anspruch 12, umfassend folgende Schritte:
i) Bereitstellen einer Mischung nach einem der Ansprüche 4, 5 oder 8 oder einer Riechstoffkomposition nach einem der Ansprüche 9 bis 11,
ii) Bereitstellen eines oder mehrerer weiterer Bestandteile des herzustellenden parfümierten Produktes, und
iii) Inkontaktbringen oder Mischen der in Schritt ii) bereitgestellten weiteren Bestandteile mit einer sensorisch wirksamen Menge der in Schritt i) bereitgestellten Bestandteile.
